# EUROPEAN PATENT APPLICATION

(11) **EP 1 504 769 A1**
(43) Date of publication of application: **09.02.2005**
(21) Application number: 03425545.5
(22) Date of filing: 08.08.2003
(51) Int. Cl.: A61L 9/04, A61K 7/32

(54) **Deodorizing or fragrancing or sanitizing product for enclosed spaces**

(71) Applicant: Deoflor S.p.A., 27030 Confienza (Pavia) (IT)
(72) Inventor: Ruffina, Laura, 27036 Mortara(prov.of Pavia) (IT); Gazzaniga Giancarlo, 27056 Salice Terme(Prov. of Pavia) (IT)
(74) Representative: Modiano, Guido, Dr.-Ing.

(57) **Abstract**

The present invention relates to a deodorizing or fragrancing or sanitizing product or the like for enclosed spaces, which comprises a solid or semisolid carrier that contains a volatile active substance. At least part of the outer surface of the carrier is provided with a coating that is constituted by a substance that adheres to the carrier. The coating contains and protects the carrier, making it easier to handle. The carrier and the coating are further preferably transparent or translucent, so as to allow to obtain particular product presentation effects based on the combination of the optical features of these components of the product.

## Description

The present invention relates to a deodorizing or fragrancing or sanitizing product or the like for enclosed spaces.

Various kinds of deodorizing or fragrancing or sanitizing product for enclosed spaces are known.

A first kind of these products substantially comprises a carrier constituted by a carrageenan gel, alginate or other gelling agents in which gradually released active volatile substances are dispersed. The gel carrier, owing to its fragility, is usually inserted in containers made of opaque or translucent synthetic material. In these kinds of products, the carrier does not collaborate substantially to the presentation of the product, since its transparency is not optimum, it is vulnerable to the touch, can be subject to syneresis, and furthermore over time tends to break up and shrink in the container, losing its initial aesthetic features.

Other kinds of products comprise a carrier based on polyolefins, which form cross-linked bonds in the presence of fragrance, producing a clear solid cross-linked structure. These carriers are generally inserted in a transparent or nontransparent solid container made of synthetic material or glass. In some cases the carrier, as the volatile active substance contained therein is gradually used up, shrinks unevenly and breaks, while in other cases wear occurs unidirectionally, maintaining the initial shape.

In other kinds of products, the carrier is constituted by waxes or urethane or polyamide polymers capable of retaining a large amount of volatile active substance, obtaining a rigid and clear product that is poured into a solid container.

The aim of the present invention is to provide a deodorizing or fragrancing or sanitizing product or the like which offers an innovative aesthetic appearance, with particular gloss effects.

Within this aim, an object of the invention is to provide a product that is rigid, protected and easy to handle even without being arranged inside a rigid container.

Another object of the invention is to provide a product in which the carrier that contains the volatile active substance cooperates decisively in achieving the aesthetic effect of the product presentation.

Another object of the invention is to provide a product that can have supporting means for its placement.

This aim and these and other objects that will become better apparent hereinafter are achieved by a deodorizing or fragrancing or sanitizing product or the like for enclosed spaces, comprising a solid or semisolid carrier that contains a volatile active substance, characterized in that at least part of the outer surface of said carrier is provided with a coating that is constituted by a substance that adheres to said carrier, said substance constituting a coating or a container of said carrier.

Further characteristics and advantages of the invention will become better apparent from the description of a preferred but not exclusive embodiment of the product according to the invention, disclosed by way of non-limitative example hereinafter.

The product according to the invention comprises a solid or semisolid carrier that contains a volatile active substance.

At least part of the outer surface of the carrier is provided with a coating, which is constituted by a substance that adheres to the carrier, constituting a coating or a container of the carrier.

The coating is preferably clear or translucent, so that the carrier is visible at least partially also in the regions affected by the coating.

The coating is any of colorless or colored and is capable of withstanding fingerprints. The color of the coating can be matched with the color of the carrier or contrast with it, depending on the aesthetic effect sought for the product.

The carrier is capable of retaining the coating and is compatible and stable with respect thereto.

The carrier can be constituted by various gelling agents; such as for example stearate, wax, gellan gum or the like, or by mixtures of aliphatic hydrocarbons, but it is preferably of the polyamide type and is capable of incorporating a considerable amount of volatile active substance, in a percentage substantially comprised between 2% and 60% with respect to the total weight of the mixture constituted by the carrier and by the volatile active substance, and is capable of retaining the volatile active substance without syneresis problems and of releasing it gradually when it is used. Preferably, the volatile active substance is present in the carrier in a percentage that is substantially comprised between 20 and 50% with respect to the total weight of the mixture constituted by the carrier and by the volatile active substance.

The carrier is preferably nonflammable or noncombustible and allows continuous release over time of the volatile active substance.

Preferably, the carrier is constituted by a polyamide resin that can have amide or ester endgroups or can be mixed with mineral oils.

Preferably, the polyamide resin is neither flammable nor combustible at the temperatures at which the product is used, has a flash point of more than 200 °C, and a closed-cup flash point of more than 94 °C. Such polyamide resin has a density of less than 1000 kg/m³ and a viscosity of approximately 770 cps at 110 °C.

The polyamide resin is preferably mixed with gelling agents, which can comprise at least one of the following substances: styrene, toluene, hexyl acetate, limonene, castor oil, mineral oils, and triglycerides.

It is also possible to use as a carrier waxes, derivatives of fatty acids, rubbers, or other substances of a known type. The carrier is in any case of the solid or semisolid type, self-supporting, preferably clear, syneresis-free and hot-workable for example by pouring into suitable molds made of silicone, metal, polycarbonate or other materials usually used to produce molds, so as to obtain the intended shape for the product being considered. Furthermore, the carrier can be divided into various quantities that are pigmented in various manners and are poured sequentially into the molds, so as to obtain a carrier that is composed of various layers that differ in terms of color and active ingredient, achieving particular multicolor effects.

Moreover, the carrier can incorporate particles or additives aimed at further improving the aesthetic appearance of the carrier. It is possible to use as additives glitter, pellets of plastic material, pearlescent colors, metallic pigments, optical brighteners, labels, et cetera.

The volatile active substance incorporated in the carrier can be constituted by a more or less complex mixture of deodorizing and/or fragrancing and/or sanitizing liquid ingredients of natural or synthetic origin. The volatile active substance can be a deodorant, a bactericidal/sanitizing substance, an insecticide or an insect repellent.

The coating is preferably impermeable and touch-resistant.

The substance that constitutes the coating has the appearance of a viscous liquid and is preferably associated with the carrier by dipping of said carrier in the substance that constitutes the coating, followed by an adequate drying period.

Optionally, the coating layer can be increased at will by means of repeated dippings of the carrier in said substance, followed by corresponding drying periods.

Furthermore, the substance that constitutes the coating can be pigmented in different manners, and the carrier can be immersed optionally in a plurality of variously pigmented coating substances in order to obtain a particular composition of the coating.

The substance that constitutes the coating is polyamide-based and is preferably constituted by a water-alcohol mixture of polyamide resins that are resistant to the colors and to the volatile active substances that are incorporated in the carrier.

Such mixture preferably has an ignition point of 370 °C (solvent), a boiling point of approximately 78 °C, a density of 940 kg/m³, a water solubility of 50% (25 °C), a vapor pressure of 58.5 millibars at 20 °C (solvent) and a vapor density of 1.6 (solvent).

The substance that constitutes the coating, moreover, can contain water in a percentage substantially comprised between 2 and 5%.

Conveniently, the product can be provided with support means, such as hooks, double-adhesive elements, or others connected to the regions of the carrier that are affected by the coating or to regions that are not affected by said coating.

Merely as an indication, examples of products according to the invention are given hereafter.

### EXAMPLE 1

| Ingredient | % |
|---|---|
| Fragrance 055200734 | 20 |
| Versagel F-1100 | 80 |
| Dye | traces |
| Sylvacote 2228E | as coating agent. |
| Versagel F-1100 is supplied by Penreco | |
| Fragrance 055200734 is supplied by Sensient Fragrances | |
| Sylvacote 2228E is supplied by Arizona Chemicals | |

Preparation: the Versagel F-1100 product, which constitutes the carrier, is heated until it fully liquefies (90 °C). The temperature is lowered to 75-80 °C and then Fragrance 055200734, which constitutes the volatile active substance, and the dye are introduced under agitation. After an appropriate mixing time, the preparation is poured into a suitable mold until it cools completely and the product solidifies accordingly. The part is removed from the mold, then the portion of the part that one wishes to coat is dipped in the solution of Sylvacote 2228E, which constitutes the coating substance. The part is left to dry for approximately 6-8 hours, then packaging is performed.

### EXAMPLE 2

| Ingredient | % |
|---|---|
| Fragrance AD9115 | 40 |
| Sylvaclear A200 | 60 |
| Dye | traces |
| Sylvacote 2228E | as coating agent. |
| Sylvaclear A200 is supplied by Arizona Chemical | |
| Fragrance AD9115 is supplied by Quest | |
| Sylvacote 2228E is supplied by Arizona Chemicals | |

Preparation: the Sylvaclear A200 product, which constitutes the carrier, is heated until it fully liquefies (60 °C), then Fragrance AD9115, which constitutes the volatile active substance, and the dye are introduced under agitation. After an appropriate mixing time, the preparation is poured into a suitable mold until it cools completely and the product solidifies accordingly. The part is removed from the mold, then the portion of the part that one wishes to coat is dipped in the solution of Sylvacote 2228E, which constitutes the coating substance. The part is left to dry for approximately 6-8 hours, then packaging is performed.

### EXAMPLE 3

| Ingredient | % |
|---|---|
| Fragrance Fiore 193 | 50 |
| Uniclear A100 | 50 |
| Dye | traces |
| Sylvacote 2228E | as coating agent. |
| Uniclear A100 is supplied by Arizona Chemical | |
| Fragrance Fiore 193 is supplied by IFF | |
| Sylvacote 2228E is supplied by Arizona Chemicals | |

Preparation: the Uniclear A100 product, which constitutes the carrier, is heated until it fully liquefies (60°C), then Fragrance Fiore 193, which constitutes the volatile active substance, and the dye are introduced under agitation. After an appropriate mixing time, the preparation is poured into a suitable mold until it cools completely and the product solidifies accordingly. The part is removed from the mold, then the portion of the part that one wishes to coat is dipped in the solution of Sylvacote 2228E, which constitutes the coating substance. The part is left to dry for approximately 6-8 hours, then packaging is performed.

In practice, the coating constituted by the polyamide substance that coats the carrier provides containment and coating of the carrier that contains the volatile active substance.

The product thus conceived is susceptible of numerous modifications and variations, all of which are within the scope of the inventive concept; all the details may further be replaced with other technically equivalent elements.

## Claims

1. A deodorizing or fragrancing or sanitizing product or the like for enclosed spaces, comprising a solid or semisolid carrier that contains a volatile active substance, **characterized in that** at least part of the outer surface of said carrier is provided with a coating that is constituted by a substance that adheres to said carrier, said substance constituting a coating or a container of said carrier.

2. The product according to claim 1, **characterized in that** said carrier is transparent and/or translucent and/or colored.

3. The product according to claims 1 and 2, **characterized in that** said carrier incorporates additives and/or particles that can be distinguished optically from the remaining part of said carrier.

4. The product according to one or more of the preceding claims, **characterized in that** said carrier is composed of a plurality of parts that can be distinguished optically from each other.

5. The product according to one or more of the preceding claims, **characterized in that** said carrier is self-supporting.

6. The product according to one or more of the preceding claims, **characterized in that** said carrier is shaped by molding in a suitable mold.

7. The product according to one or more of the preceding claims, **characterized in that** said carrier is constituted by gelling agents such as stearate, wax, gellan gum.

8. The product according to one or more of the preceding claims, **characterized in that** said carrier is constituted by mixtures of aliphatic hydrocarbons.

9. The product according to one or more of the preceding claims, **characterized in that** said carrier is constituted by a polyamide resin.

10. The product according to one or more of the preceding claims, **characterized in that** said polyamide resin has ester or amide endgroups or is mixed with mineral oils.

11. The product according to one or more of the preceding claims, **characterized in that** said polyamide resin is mixed with other gelling agents.

12. The product according to one or more of the preceding claims, **characterized in that** said volatile active substance is present in said carrier in a percentage that is substantially comprised between 2 and 60% by weight with respect to the total weight of the mixture constituted by the carrier and by the volatile active substance.

13. The product according to one or more of the preceding claims, **characterized in that** said volatile active substance is present in said carrier in a percentage substantially comprised between 20 and 50% by weight with respect to the total weight of the mixture constituted by the carrier and by the volatile active substance.

14. The product according to one or more of the preceding claims, **characterized in that** said volatile active substance is constituted by a deodorant and/or by a fragrance and/or by a bactericidal/sanitizing substance and/or by an insecticide and/or by an insect repellent.

15. The product according to one or more of the preceding claims, **characterized in that** said coating is impermeable.

16. The product according to one or more of the preceding claims, **characterized in that** said coating is clear or translucent.

17. The product according to one or more of the preceding claims, **characterized in that** said coating is associated with said carrier by dipping said carrier in said substance that constitutes said coating in the liquid state, followed by a period for drying said substance.

18. The product according to one or more of the preceding claims, **characterized in that** the substance that constitutes said coating, when applied to said carrier, is liquid and is constituted by a water-alcohol mixture of polyamide resins.

19. The product according to one or more of the preceding claims, **characterized in that** said water-alcohol mixture of polyamide resins is stable with respect to the formulation of said active substance.

20. The product according to one or more of the preceding claims, **characterized in that** it comprises supporting means that are connected to regions of said carrier that are affected by said coating or to regions of said carrier that are not affected by said coating.
